Europäisches Patentamt

European Patent Office

Office eurcpéen des brevets

(19)

(11) Publication number:

**0 383 595**
**A1**

## EUROPEAN PATENT APPLICATION

(12)

(21) Application number: **90301616.0**

(22) Date of filing: **15.02.90**

(51) Int. Cl.⁵: **A61L 27/00**

(30) Priority: **16.02.89 GB 8903565**

(43) Date of publication of application:
**22.08.90 Bulletin 90/34**

(84) Designated Contracting States:
**GR**

(71) Applicant: **NATIONAL RESEARCH DEVELOPMENT CORPORATION**
**101 Newington Causeway**
**London SE1 6BU(GB)**

(72) Inventor: **Graham, Neil Bonnette**
**6 Kilmardinny Grove**
**Bearsden, Glasgow G61 3NY(GB)**

(74) Representative: **Hamilton, Raymond**
**Patent Department National Research Development Corporation 101 Newington Causeway**
**London SE1 6BU(GB)**

(54) **Cement compositions.**

(57) The incorporation of cross linked polymeric particles derived from a vinyl or vinylidene group containing monomer especially an ester of acrylic or methacrylic acid into the liquid phase of a two phase self polymerisable resin cement has been found to be advantageous. It reduced the exotherm produced on mixing the solid and liquid phase components and the degree of shrinkage upon setting. The novel cements find application as dental cements and more especially as bone cements.

EP 0 383 595 A1

## CEMENT COMPOSITIONS

This invention relates to novel acrylic resin based cements and in particular bone cements which are useful in the sealing of intraosseous prostheses.

Resin based cements find use in a variety of applications. One area of application is within the body both in the implantation of protheses of various human joints such as the knee, elbow, shoulder and hip joints and also as dental cements. These cements must be tolerated by the body and should preferably be easily manipulated during surgery or dental treatment and have good mechanical properties when set. The conventional cements are supplied as a two component system; a liquid phase component comprising a methacrylate monomer and a solid phase component (usually a powder) comprising polymethyl methacrylate, which components are mixed immediately prior to use. After mixing the two components form an oily paste-like kneadable mass which can be introduced into the bone cavity. The mixture then polymerises in-situ which brings about the setting of the cement within a relatively short period, say 15 minutes.

Although such cements are in widespread use, it is recognised that they do suffer from some disadvantages. One such disadvantage is due to the fact that the polymerisation is a highly exothermic reaction which can raise the local temperature to undesirably high levels, sometimes as high as $100^\circ$ C. This has been found to be particularly disadvantageous in the implantation of protheses. Another problem in utilising these resin based compositions is the shrinkage which occurs during the polymerisation process.

British Patent Application 2017732 describes an acrylic bone cement which utilises a liquid phase which comprises a collodion of an acrylic polymer or copolymer in at least one monomer. These liquid phases are formed by dissolving approximately 1 part by weight of a polymethyl methacrylate in approximately 2 parts by weight of methyl methacrylate. These systems do reduce the exotherm generated by the polymerisation reaction to some degree but their utilisation is further complicated because the liquid phase is relatively high viscous, typically around 500 centipoise at $20^\circ$ C which renders it difficult to mix the liquid and solid phases efficiently.

This invention provides an improved acrylic cement which utilises a novel liquid phase which is substantially less viscous and thereby easier to mix with the solid phase and which substantially reduces the exotherm associated with the polymerisation reaction. The compositions of this invention may also exhibit a lower degree of shrinkage. From one aspect our invention provides a self polymerisable cement which is prepared by the intimate admixture of a solid phase component comprising an acrylic polymer or copolymer and a liquid phase component comprising an acrylic monomer which is characterised in that the liquid phase further comprises a polymeric material comprising cross-linked particles of a homo or copolymer of a substituted or unsubstituted vinyl or vinylidene group containing monomer which monomer is susceptible to free radical addition polymerisation cross-linked by a bis, tris or higher vinyl or vinylidene group containing monomer which particles are capable of forming a sol in a solvent which (a) has a solubility parameter of from 2.5 cal$^{1/2}$ ml$^{-3/2}$ below to 2.5 cal$^{1/2}$ ml$^{-3/2}$ above the solubility parameter of the material produced by bulk polymerising the monomer or monomer mixture and (b) is of the same or adjacent hydrogen bonding group as the material produced by bulk polymerising the monomer or monomer mixture and which particles are produced by polymerisation of the monomer and the crosslinking monomer in such solvent in a manner which avoids gelation of the polymer.

These polymeric materials will hereinafter be termed for convenience "microgels". They are preferably prepared by the processes which are described in our earlier British Patent 2090264. The preferred microgels are those which utilise methacrylate esters especially the lower alkyl esters, i.e. methyl, ethyl, propyl, or butyl or 2-ethylhexyl methacrylate as the homo or copolymer. Any copolymerisable monomer unit which is greater than bifunctional may be utilised as the crosslinking agent. Examples of monomers which may be employed to advantage are unsubstituted or hydro-carboxylated aromatic hydrocarbons such as divinyl benzene and the polyallyl substituted esters of poly(carboxylic) benzenes such as allyl esters of phthalic acid or pyromellitic acid. Preferably the cross linking agent comprises a bis or tris vinyl or vinylidene group containing ester such as the bis methacrylate ester of polyethylene glycol, the bis methacrylate ester of ethylene glycol or triallyl cyanurate The cross-linked particles preferably have a weight average molecular weight of from 5000 to 20,000,000. The dispersed particles preferably have a mean particle size in the range 10nm to 1μm preferably in the range 10nm to 0.1μm.

The solvent used in the processes for the preparation of the acrylate microgels may qualitatively be assigned to one of three groups according to its hydrogen bonding capacity:

| GROUP | EXAMPLES |
|---|---|
| strongly hydrogen bonded | alcohols, acids, amines, aldehydes |
| moderately hydrogen bonded | ketones, esters, ethers, |
| poorly hydrogen bonded | hydrocarbons, chlorinated and nitrohydrocarbons, nitriles |

from which the meaning of "same or adjacent hydrogen bonding group" will be apparent.

The solubility parameter (which is the square root of the cohesive energy density) of a number of solvents are listed in our earlier British Patent 2090264 as are the solubility parameter ranges for a number of bulk acrylic polymers. These parameters may also be ascertained from any standard work of reference e.g. "Encyclopaedia of Polymer Science and Technology" published by John Wiley & Sons. The preferred solvents for use in the preparation of the microgels are those which have a solubility parameter up to 1.0 $cal^{1/2}$ $ml^{-3/2}$ above the solubility parameter of the material produced by bulk polymerising the monomer or monomer mixture.

In practice, an appropriate solvent for the production of a microgel useful in the compositions of this invention may readily be selected by first bulk polymerising the monomer mixture to a solid mass and then determining the swellability of portions of that mass in a number of solvents. Those solvents which swell it to a significant degree and preferably a degree which is in excess of 100 pph of polymer will generally be suitable for the production of the microgels which are useful in the compositions of the present invention, provided that they do not contain reactive groups which will interfere with the polymerisation. Those solvents which impart the greatest degree of swelling are preferred. To obtain high molecular weight cross-linked particles which are capable of forming sols in the solvent, it is desirable to maintain a thermodynamically good solvent at all stages of the reaction.

In the majority of cases the reaction will proceed to 100% conversion of monomer to polymer without any visible sign of gelation. In some instances, e.g. where the polymerisation is carried out using a higher concentration of monomer or a less thermodynamically good solvent, it may be preferable or necessary to monitor the progress of the polymerisation in order to avoid gelation. One monitoring method which may be employed is to spread an aliquot of the reaction mixture onto a glass slide and to observe whether or not a coherent film is formed. When such a film is formed or when other monitoring suggests the polymerisation may be terminated into to avoid the possibility of gelation. Termination can be brought about in any conventional manner, e.g. by adding a polymerisation quenching agent or by reducing the temperature of the reaction medium.

At the end of the reaction the crosslinked polymer particles may conveniently be separated from the reaction medium by mixing with an excess of a non-solvent. Alternatively the particulate solids can be separated by solvent evaporation. Separation by addition or reverse addition to a non-solvent such as petroleum ether or hexane is generally preferred.

Liquid phase components which comprise an acrylic monomer and a microgel as hereinbefore defined are believed to be novel and comprise a further aspect of this invention. The liquid phase preferably comprises the monomer and microgel components in a weight ratio of from 3:1 to 10:1 preferably 4:1 to 6:1. Incorporation of larger proportions of microgel increases the viscosity of the solution to undesirable levels whereas incorporation of lower proportions of microgel has a proportionately decreased effect upon the exotherm during the polymerisation step.

The acrylic monomers used in the liquid phase are those which are generally known to be useful in the art. The esters of acrylic and methacrylic acid are preferred for present use especially the methyl, ethyl, propyl and butyl acrylates and methacrylates. The liquid phase of the compositions of this invention may also comprise other conventional additives such as polymerisation catalysts and cross linking agents. The presence of a polymerisation catalyst in the cement mixture is strongly preferred. These catalysts are well known in the art. A preferred group of catalysts are the peroxides especially benzoyl peroxide and percarbonates to which tertiary amines such as NN dimethyl paratoluidine are added. In a preferred embodiment one component of such a binary catalyst system is added to the liquid phase and the other to the solid phase. The liquid phase of the compositions of this invention preferably comprise such a tertiary amine. The solid phases preferably comprise a peroxide or percarbonate.

The microgel is readily dispersable in the monomer and the liquid phase component may readily be prepared by simple addition of the microgel to the monomer. These liquid phases normally have a viscosity of less than 50 centipoise and more usually less than 25 centipoise. The combination of small particle size and low viscosity exhibited by the liquid phase is particularly advantageous in that it may be passed through conventional filters as part of a sterilisation process.

The viscosity of the liquid phase may increase upon prolonged storage even at low temperatures. We have discovered that this tendency to instability can be reduced by the addition of a polymerisation inhibitor such as hydroquinone. Liquid phase comprising such an inhibitor form a preferred aspect of the present invention.

The solid phase may be formulated in a conventional manner. It will comprise an acrylic polymer or copolymer which is preferably polymethyl methacrylate optionally in admixture with other conventional additives. The polymer may be in the form of a powder or fine granules. The size of the polymer particles is not crucial and may be as high as 500 microns. The size of the particles may have an affect upon the setting time of the cement and this may be varied according to the intended application of the cement. The solid phase may also contain a mineral charge such as alumina or zirconium oxide. Other typical additives which may usefully be incorporated into the solid phase include x-ray opacifiers such as barium sulphate, a polymerisation initiator particularly a free radical initiator, a colouring agent and antibiotic agents to control the risk of infection. The solid and liquid phases are packaged separately normally in sterile containers. The quantities will be such as to enable the user to mix the contents of the packs to obtain a paste which is ready for application. A pack comprising two such separate packages forms a further aspect of this invention.

The solid and liquid phases are mixed in the conventional proportions. In the case of cement compositions designed to be used in the sealing of intraosseous prostheses (bone cements) quantities that provide approximately two parts by weight of the solid phase to one part by weight of the liquid phase may conveniently be employed. These proportions may be varied but generally the weight ratio of solid phase to liquid phase will be within the range 1:1 to 3:1 and more usually within the range 1.5:1 to 2.5:1.

Mixing of the solid and liquid phase components may be readily achieved by manual or mechanical manipulation. We have discovered that the homogenicity of the mixed but unset cement can be further improved by the addition of a solvent especially a polar solvent such as ethanol. This solvent may conveniently be added to the liquid phase prior to mixing. Where the mechanical strength of the set cement is important it may be useful to incorporate small quantities of fibrous materials such as carbon fibres, acrylic fibres, polyamide or polyester fibres.

The invention is illustrated by the following examples.

Example 1

An acrylate microgel was prepared by mixing the following ingredients:

1465gms methyl methacrylate
32.67gms ethylene dimethacrylate and
60.71gms benzoyl peroxide were reacted by refluxing in 15 litres of methyl ethyl ketone for 24 hours.

The solution was brought to its "haze point", by the slow addition of about 55% of its volume, of petrol ether 100-120. (This is the point when the solution just turned permanently turbid.)

The acrylate microgel was precipitated by running the solution brought to its haze point, steadily under stirring, into 450% of its volume of petrol ether 100-120.

The slurry was filtered, washed with fresh petrol ether and then dried in a vacuum oven.

Approximately 1290gms of microgel were obtained.

The acrylate microgel was mixed with a proprietory methyl methacrylate monomer containing 1.81% w/w N:N-dimethyl-p-toluidine, 0.22% w/w ascorbic acid and 0.002% w/w hydroquinone in various proportions as set out in Table 1. These mixtures were combined with a proprietary polymethyl methacrylate powder. The mixture was continuously stirred/ The time taken to form a dough (i.e. a mixture which did not adhere to an instrument dipped into it) was recorded.

The dough was shaped into an oval ball. An aluminium foil covered thermistor of a digital thermometer was inserted into the centre of the dough mass. The dough mass was placed in a foamed polystyrene mould. The temperature of the mass was recorded against time until the thermometer registered 37°C. The setting time was recorded as the time measured from the beginning of the mixing until temperature of the dough reaches a value of

$$\underline{\frac{Tamb + Tmax}{2}}$$

where Tmax is the maximum temperature and Tamb is the ambient temperature.

The results are set out in Table 1.

## Example 2

A comparison was carried out between a commercial bone cement formulation A and a composition of this invention B. The commercial product comprised a solid phase component which was a methyl methacrylate polymer and a liquid phase comprising 98% by weight of methyl methacrylate and 2% by weight of dimethyl p toluidine. The composition according to the invention was produced by replacing 15% by weight of the methacrylate with the acrylate microgel produced according to the process of Example 1.

The cements were mixed in the proportions shown in the following table. The maximum hardening temperature was determined according to ISO 5833/1 and the Impact Strength and flexural strength of the set cement was determined according to DIN standards. The results are set out in the following table.

| | Formulation A | Formulation B |
|---|---|---|
| Ratio | | |
| Powder (gm):Liquid(ml) | 40:20 | 40:20 |
| Impact Strength KJ/m$^2$ | 6:30 | 6:40 |
| Flexural Strength N/m$^2$ | 85.9 | 95.0 |
| Maximum Temperature $^\circ$C | 73.0 | 63.5 |

## TABLE 1

| POWDER | LIQUID | DOUGH MINS | SET MINS | S - D MINS | EXOTHERM TEMP $^\circ$C |
|---|---|---|---|---|---|
| Proprietary PMMA (40 gm) + 4.0 gram BaSO$_4$ | Proprietary MMA (20 gm) | 1.5 | 8.75 | 7.25 | 106.7 |
| Proprietary PMMA (40 gm) + 4.0 gram BaSO$_4$ | Proprietary MMA with 3.0 gram replacement by microgel + 1.0 gram Ethanol | 1.0 | 6.75 | 5.75 | 97.1 |
| Proprietary PMMA (40 gm) + 4.0 gram BaSO$_4$ | MMA with 2.5 gram replacement by microgel + 1.0 gram Ethanol | 1.5 | 7.25 | 5.75 | 93.7 |
| Proprietary PMMA (- 2.0 grams) + 4.0 gram BaSO$_4$ | MMA 16 grams with 3.0 gram microgel + 1.0 gram Ethanol | 1.0 | 7.5 | 6.5 | 95.2 |
| Proprietary PMMA + 4.0 gram BaSO$_4$ | MMA 16 gram with 2.5 gram microgel + 1.0 gram A Ethanol | 1.25 | 6.25 | 5.0 | 96.5 |
| * Digital Thermometer - Thermistor | | | | | |
| + Pen Chart Recorder - Copper-Constantin Thermocouple | | | | | |
| ** Formulations selected for improved wettability and exotherm. | | | | | |

## Claims

1. A liquid polymeric composition useful as one component of a self polymerisable cement comprising an acrylic monomer which is characterised in that it further comprises a polymeric material comprising cross linked particles of a homo- or co-polymer of a substituted or unsubstituted vinyl or vinylidene group containing monomer which monomer is susceptible to free radical addition polymerisation, cross linked by bis or tris or higher vinyl or vinylidene group containing monomer which particles are capable of forming a sol in a solvent which (a) has a solubility parameter of from 2.5 $cal^{\frac{1}{2}}$ $ml^{-3/2}$ below to 2.5 $cal^{\frac{1}{2}}$ $ml^{-3/2}$ above the solubility parameter of the material produced by bulk polymerising the monomer or monomer mixture and (b) is of the same or adjacent hydrogen bonding group as the material produced by bulk polymerising the monomer or mixture and which particles are produced by polymerising the monomer(s) and the cross linking agent in a manner which avoids gelation of the polymer.

2. A polymeric composition according to claim 1 characterised in that the cross linked particles are derived from the polymerisation of a methacrylate ester.

3. A polymeric composition according to claim 2 characterised in that the methacrylate ester is selected from the group comprising methyl, ethyl, propyl, butyl or 2-ethylhexyl methacrylates.

4. A composition according to any of the preceding claims characterised in that the cross linking agent is selected from the group comprising the bis methacrylate ester of polyethylene glycol, the bis methacrylate ester of ethylene glycol and triallyl cyanurate.

5. A composition according to any of the preceding claims characterised in that the cross linked particles have a weight average molecular weight of from 5,000 to 20,000,000.

6. A composition according to any of the preceding claims characterised in that the cross linked particles have a mean particle size in the range 10 nm to 0.1 $\mu$m.

7. A composition according to any of the preceding claims characterised in that the acrylic monomer is selected from the group comprising methyl, ethyl, propyl and butyl acrylates and methacrylates.

8. A composition according to any of the preceding claims characterised in that it comprises the monomer and cross linked particle components in a weight ratio of from 3:1 to 10:1.

9. A composition according to any of the preceding claims characterised in that it has a viscosity of less than 50 centipoise.

10. A composition according to any of the preceding claims characterised in that it further comprises a polar solvent.

11. A composition according to any of the preceding claims characterised in that it further comprises a polymerisation inhibitor.

12. A composition according to claim 11 characterised in that the polymerisation inhibitor is a hydroquinone.

13. A composition according to any of the preceding claims characterised in that the liquid comprises one component of a binary polymerisation catalyst system.

14. A composition according to claim 13 characterised in that the liquid phase comprises a tertiary amine.

15. A self polymerisable sealing cement which is prepared by the intimate admixture of a solid phase component comprising an acrylic acid polymer or copolymer and a liquid phase component according to any of claims 1 to 14.

16. A pack for preparing a self polymerisable sealing cement according to claim 15 characterised in that it comprises the solid phase component and the liquid phase component in separate sterile packs.

17. A polymeric composition according to any of claims 1 to 14 substantially as hereinbefore described with reference to the foregoing examples.

18. A self polymerisable sealing cement substantially as hereinbefore described with reference to the foregoing examples.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | GB-A-2 017 732 (S.E.P.C., SOCIETE D'EXPLOITATION DES PROCEDES COATEX) * Claims; examples; page 2, line 61 - page 3, line 29 * | 1-18 | A 61 L 27/00 |
| Y,D | GB-A-2 090 264 (NATIONAL RESEARCH DEVELOPMENT CORP.) * Claims; page 20, lines 13-46 * | 1-18 | |
| A | EP-A-0 080 405 (ALTULOR) | | |
| A | WO-A-8 602 370 (FERRING LABORATORIES) | | |

TECHNICAL FIELDS
SEARCHED (Int. Cl.5)

A 61 L
A 61 K
C 08 F

The present search report has been drawn up for all claims

| Place of search | Date of completion of the search | Examiner |
|---|---|---|
| THE HAGUE | 16-05-1990 | COUSINS-VAN STEEN G.I.L. |